(19) Europäisches Patentamt
European Patent Office
Office européen des brevets

(11) EP 3 699 219 A1

(12) # EUROPÄISCHE PATENTANMELDUNG

(43) Veröffentlichungstag:
**26.08.2020 Patentblatt 2020/35**

(51) Int Cl.:
***C08G 18/79*** *(2006.01)* ***C09D 175/00*** *(2006.01)*
***C08G 18/38*** *(2006.01)* ***C07C 227/06*** *(2006.01)*

(21) Anmeldenummer: **19158880.5**

(22) Anmeldetag: **22.02.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **NEUE ZWEIKOMPONENTEN-KLARLACKSYSTEME ENTHALTEND POLYASPARAGINSÄUREESTER**

(57) Die vorliegende Erfindung betrifft Zweikomponenten- Klarlacksysteme enthaltend Polyasparaginsäureester mit primären Aminogruppen und geringe Mengen an Fumarsäuredialkylester, ein Verfahren zu deren Herstellung und deren Einsatz zur Herstellung von Beschichtungen für Fahrzeugreparaturanwendungen und damit beschichtete Substrate.

EP 3 699 219 A1

## Beschreibung

Die vorliegende Erfindung betrifft Zweikomponenten-Klarlacksysteme enthaltend Polyasparaginsäureester mit primären Aminogruppen und geringe Mengen an Fumarsäuredialkylester, ein Verfahren zu deren Herstellung und deren Einsatz zur Herstellung von Beschichtungen für Fahrzeugreparaturanwendungen und damit beschichtete Substrate.

Zweikomponenten (2K)-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente enthalten, sind seit langem bekannt. Sie eignen sich zur Herstellung von hochwertigen Überzügen, die hart, elastisch, abrieb- und lösemittelbeständig und vor allem auch witterungsstabil eingestellt werden können.
Innerhalb dieser 2K-Polyurethan-Beschichtungstechnologie haben sich in letzter Zeit bestimmte, estergruppenhaltige, sekundäre Polyamine etabliert, die sich in Kombination mit Lackpolyisocyanaten insbesondere als Bindemittel in lösemittelarmen oder -freien (high solids) Beschichtungsmitteln eignen und eine rasche Aushärtung der Beschichtungen bei niedrigen Temperaturen, wie in Fahrzeugreparaturanwendungen erforderlich ist, ermöglichen.

Bei diesen sekundären Polyaminen handelt es sich um die sogenannten Polyasparaginsäureester, wie sie beispielhaft in der EP0403921 beschrieben werden. Ihre Verwendung allein oder in einem Gemisch mit weiteren, gegenüber Isocyanatgruppen reaktionsfähigen Komponenten in 2K-Beschichtungsmitteln wird beispielsweise in der EP0403921, EP0639628, EP0667362, EP0689881, US5214086, EP0699696, EP0596360, EP0893458, DE19701835 und US5243012 beschrieben. EP0470461 beschreibt eine Beschichtung für Fahrzeugreparaturanwendungen, die eine Polyisocyanatkomponente und ein gegenüber Isocyanat reaktives sekundäres Diamin, hergestellt aus 3,3'-Dimethyl-4,4'-diaminodicyclohexylmethan und Maleinsäurediethylester enthält. Die isocyanatreaktive Komponente enthält weiterhin eine hydroxylfunktionelle Komponente von Polyhydroxypolyacrylate oder Mischungen von Polyhydroxypolyacrylaten und Polyesterpolyolen. WO15130501 und WO15130502 offenbaren Polyasparaginsäureester-Zusammensetzungen, die zwischen 15 und 30% Asparaginsäureester mit primären Aminogruppen (gemessen als Flächen-% im Gas Chromatogram) aufweisen. In beiden Schriften wird jedoch kein Vorteil aufgrund eines erhöhten Gehaltes an Asparaginsäureester mit primären Aminogruppen erkannt und Polyasparaginsäureester-Zusammensetzungen mit akzeptabler Topfzeit konnten nur durch eine weitere Umsetzung mit bevorzugt cycloaliphatischen Polyisocyanaten erreicht werden. Es ist anzuhnemen, dass die so hergestellten Beschichtungsmittel aufgrund des herkömmlichen Herstellverfahren keinen reduzierten Gehalt an Fumarsäurediethylester aufweisen.

Die Herstellung von aminofunktionellen Asparaginsäureestern ist im Prinzip bekannt. Die Synthese erfolgt über eine Addition von primären Polyaminen an eine aktivierte Kohlenstoff-Doppelbindung vinyloger Carbonylverbindungen, wie beispielsweise in Malein- oder Fumarsäureestern enthalten, die in der Literatur hinreichend beschrieben ist (Hauben Weyl, Meth. d. Org. Chemie Bd. 11/1, 272 (1957), Usp. Khim. 1969, 38, 1933). Bei dieser Reaktion kann es zur Bildung eines Polyasparaginsäureester mit primären Aminogruppen als Nebenprodukt kommen, wenn nur eine Aminogruppe des Polyamins mit der Doppelbindung der vinylogen Carbonylverbindungen reagiert hat. In den kommerziell erhältlichen Polyasparaginsäureestern wird Maleinsäureester als die vinyloge Carbonylverbindung eingesetzt. Während der Herstellung eines Polyasparaginsäureester auf Basis von Maleinsäureester kann eine retro-Michael Addition als eine weitere unerwünschte Nebenreaktion erfolgen, bei der durch Eliminierung des Polyamins als Nebenkomponente Fumarsäuredialkylester gebildet wird. Ein typisches Herstellverfahren eines Polyasparaginsäureesters erfordert daher eine Lagerzeit von 4-6 Wochen nachdem der Hauptteil der Edukte miteinander reagiert hat. In dieser Zeit erfolgt die sogenannte Reifung des Produktes, welche sich durch Stabilisierung der Viskosität manifestiert. Dadurch, dass der Umsatz innerhalb dieser Zeit weiter steigt, sinkt auch der Gehalt an Fumarsäuredialkylester. Diese Lagerung über mehrere Wochen führt zu erheblichen Logistik-Kosten innerhalb der Produktion. Obwohl das Produkt erst nach der Lagerung an den Kunden ausgeliefert wird, enthält es immer noch substantielle Mengen Fumarsäuredialkylester welches eine starke Sensibilisierung verursachen kann. Fumarsäurediethylester ist beispielsweise als VOC (volatile organic compounds) klassifiziert und verhindert damit eine Bereitstellung VOC-freier Beschichtungen. Ein weiterer Nachteil, der durch die Anwesenheit von Fumarsäuredialkylester hervorgerufen wird, ist die Erniedrigung der Glassübergangstemperatur eines Lackfilmes durch seine weichmachende Wirkung.
Es bestehen theoretisch zwei Möglichkeiten das Verfahren zur Herstellung von Polyasparaginsäureester-Zusammensetzungen zu ändern, damit Polyasparaginsäureester-Zusammensetzungen entstehen, die die oben genannten Nachteile nicht aufweisen. Die Reaktionszeit kann verlängert oder die Reaktionstemperatur erhöht werden. Ersteres scheidet aus ökonomischen Gründen aus. Eine Erhöhung der Reaktionstemperatur auf z.B. 100°C oder sogar auf 80°C führt wiederum zu einer drastischen Gelbfärbung des Produktes.

EP0816326 offenbart ein Verfahren zur Beschleunigung der Addition des Polyamins an Dialkylmaleinat bzw. zum Reduzieren des Fumarsäuredialkylesters durch die Zugabe eines speziellen Katalysators. Da trotz des Einsatzes eines Katalysators die Notwendigkeit einer Lagerung nicht verhindert werden kann, führt dieser Lösungsansatz nicht zum endgültig zufriedenstellenden Ergebnis. In EP1197507 ist die Zugabe von Thiol-Verbindungen als Fänger für Fumarsäuredialkylester beschrieben. Dadurch, dass die Thiol-Verbindingen bekanntermaßen eine erhebliche Geruchsbelästigung verursachen ist auch diese Lösung nicht in der Praxis umsetztbar.

Eine theoretische Möglichkeit des destillativen Aufarbeitens ist z.B. in der EP0403921 erwähnt. Es handelt sich dabei um ein destillatives Entfernen von Fumarsäuredialkylester innerhalb eines Verfahrens, bei dem ein Überschuß von Dialkylmaleinat eingesetzt wird. Diese Offenbarung gibt keine Beispiele und auch kein Verfahren zur Destillation an. Da in diesem Verfahren ein Überschuss an Diethylmaleinat eingesetzt wird ist nicht zu erwarten, dass das aufgearbeitete Produkt deutliche Mengen an Polyasparaginsäureestern mit primären Aminogruppen aufweist. Dieses Verfahren hat sich nicht durchgesetzt, da die eingesetzte überschüssige Menge an Diethylmaleinat zu einer schlechten Zeit-Raum-Ausbeute und viel Abfall führt, was ökonomisch nicht vertretbar ist.

DE102006002153 beschreibt ebenfalls ein Produkt welches unter Einsatz eines Überschusses Dialkylmaleinat und anschließende destillative Entfernung von Fumarsäuredialkylester hergestellt wird. Es handelt sich um einen Diasparaginsäureester, der frei von primären Aminogruppen ist.

WO2018/074884, welche zum Zeitpunkt der Anmeldung der vorliegenden Erfindung zum Patent noch nicht veröffentlicht war, beschreibt ebenfalls die Destillation von Polyasparaginsäureestern und ganz allgemein deren Verwendung in Beschichtungsmitteln. Klarlacke enthaltend zwingend Wasserfänger und/oder Hydrolyseschutzmittel werden nicht beschrieben.

Die in dem Stand der Technik offenbarten Beschichtungszusammensetzungen auf Polyasparaginsäureesterbasis weisen schnelle Aushärtezeiten bei ausreichender Topfzeit aus und zeichnen sich durch gute mechanische und optische Eigenschaften aus. Es ist allerdings dem Fachmann bekannt, dass solche Beschichtungszusammensetzungen unter dem Einfluss von Feuchtigkeit zu Mängeln in der Adhäsion und Zwischenschichthaftung in einem Mehrschichtstruktur-Aufbau neigen. In der Fahrzeugreparatur-Praxis erfordert diese Empfindlichkeit gegen Feuchte verlängerte Lagerungszeiten von den lackierten Teilen bevor die an einen Fahrzeug eingebaut werden können.

Die Aufgabe der vorliegenden Erfindung besteht somit darin, eine Beschichtungszusammensetzung auf Polyasparaginsäureesterbasis mit einer ausgewogenen Trocknungsleistung, d.h. mit schnellen Aushärtezeiten bei einer ausreichenden Topfzeit, und verbesserter Wasserbeständigkeit bereit zu stellen.

Die Aufgabe der vorliegenden Erfindung konnte durch die Bereitstellung von zweikomponentigen Beschichtungszusammensetzungen (2K-Beschichtungsmittel), enthaltend Polysparaginsäureester mit primären Aminogruppen und geringen Mengen an Fumarsäuredialkylester, gelöst werden.

Gegenstand der vorliegenden Erfindung sind zweikomponentige Beschichtungszusammensetzungen (2K-Beschichtungsmittel), enthaltend

a) mindestens eine Polyasparaginsaureester-haltige Komponente A,
b) mindestens eine Polyisocyanatkomponente B
c) gegebenenfalls eine oder mehrere, von A verschiedene, gegenüber Isocyanatgruppen reaktionsfähige Komponenten C,
d) mindestens ein Wasserfänger und/oder Hydrolyseschutzmittel (Komponente D1) und gegebenenfalls weitere Hilfs- und Zusatzstoffe (Komponente D2).

Im Rahmen der vorliegenden Erfindung sind Polyasparaginsaureester-haltige Komponenten A Zusammensetzungen enthaltend einen oder mehrere Polyasparaginsäureester der allgemeinen Formel (I)

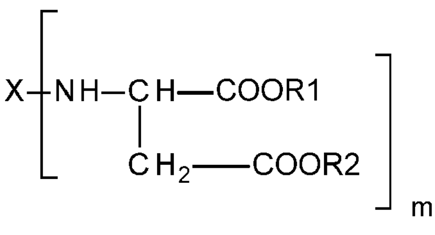

(I),

in welcher

X für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,

R1 und R2 für gleiche oder verschiedene organische Reste, mit je 1 bis 18 Kohlenstoffatomen stehen,

m für eine ganze Zahl >1 steht,

und

einen oder mehrere Polyasparaginsäureester mit primärer Aminogruppe der allgemeinen Formel (II)

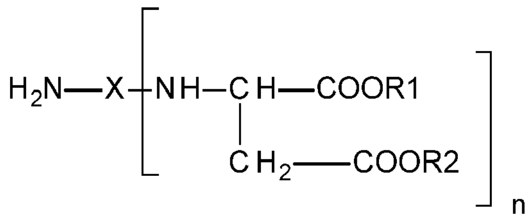

(II)

in welcher

n für m-1 steht,

X, Reste R1 und R2 die oben genannten Bedeutungen haben,

dadurch gekennzeichnet, dass der Anteil der Verbindungen der allgemeinen Formel (II) von 1% bis 20% der GC Oberfläche (gemessen als Flächen-% im Gas Chromatogram) entspricht, wobei die Summe der GC Oberflächen der Verbindungen der beiden allgemeinen Formeln (I) und (II) 100% beträgt und in Komponente A Fumarsäuredialkylester in Mengen von 0.01 bis 1,2 Gew.-%, bezogen auf das Gesamtgewicht der Komponente A, vorliegen.

Bevorzugt sind Polyasparaginsaureester-haltige Komponenten A Zusammensetzungen enthaltend einen oder mehrere Polyasparaginsäureester der allgemeinen Formeln (I) und (II) bei denen R1 und R2 für gleiche oder verschiedene Alkylreste mit je 1 bis 18 Kohlenstoffatomen, bevorzugt gleiche oder verschiedene Alkylreste mit je 1 bis 8 Kohlenstoffatomen und ganz besonders bevorzugt für jeweils Alkylreste wie Methyl-, Ethyl-, Propyl-, iso-Propyl-, Butyl- oder iso-Butyl-Reste stehen. Am meisten bevorzugt ist Ethyl.

Polyasparaginsaureester-haltige Komponenten A sind Zusammensetzungen enthaltend einen oder mehrere Polyasparaginsäureester der allgemeinen Formeln (I) und (II), bei denen X für organische Reste steht, die durch Entfernung der primären Aminogruppen aus einem entsprechenden, (cyclo)aliphatisch bzw. araliphatisch gebundene primäre Aminogruppen aufweisenden Polyamin erhalten werden, ausgewählt aus der folgenden Gruppe: alle bekannten Polyamine mit primären Aminogruppen, die der allgemeinen Formel (III) entsprechen. Beispielhaft seien die folgenden Verbindungen genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (Dytek®A, Fa DuPont), 1,6-Diaminohexan, 2,2,4- und/oder 2,4,4-Trimethyl-1,6-diaminohexan, 1,11-Diaminoundecan, 1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, amino-funktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Bevorzugt sind Polyasparaginsaureester-haltige Komponenten A Zusammensetzungen enthaltend einen oder mehrere Polyasparaginsäureester der allgemeinen Formeln (I) und (II) bei denen X für organische Reste steht, die durch Entfernung der primären Aminogruppen aus einem der Polyamine der allgemeinen Formel (III) erhalten wird, worin m = 2 bedeutet und X für einen cyclischen Kohlenwasserstoffrest mit mindestens einem cyclischen Kohlenstoffring steht. Beispiele für besonders bevorzugt einsetzbare Diamine sind 1-Amino-3,3,5-trimethyl-5-aminomethylcyclohexan (IPDA), 2,4- und/oder 2,6-Hexahydrotoluylendiamin (H6-TDA), Isopropyl-2,4-diaminocyclohexan, und/oder Isopropyl-2,6-diaminocyclohexan, 1,3-Bis-(aminomethyl)-cyclohexan, 2,4'-, und/oder 4,4'-Diamino-dicyclohexylmethan, 3,3'-Dimethyl-4,4'-diamino-dicyclohexylmethan (Laromin® C 260, Fa. BASF AG), die isomere, eine Methylgruppe als Kernsubstituenten aufweisende Diaminodicyclohexylmethane (=C-Monomethyl-diaminodicyclohexylmethane), 3(4)-Aminomethyl-1-methylcyclohexylamin (AMCA) sowie araliphatische Diamine, wie z.B. 1,3-Bis-(aminomethyl)-benzol oder m-Xylylendiamin.

Ebenfalls bevorzugt sind Polyasparaginsaureester-haltige Komponenten A Zusammensetzungen enthaltend einen oder mehrere Polyasparaginsäureester der allgemeinen Formeln (I) und (II) bei denen X für organische Reste steht, die durch Entfernung der primären Aminogruppen aus einem der Polyamine der allgemeinen Formel (III) erhalten wird, ausgewählt aus der Gruppe: Polyetherpolyamine mit aliphatisch gebundenen primären Aminogruppen, 1,2 Diaminopropan, 1,4-Diaminobutan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 1,5-Diaminopentan, 2,4'- und/oder 4,4'-Diamino-dicyclohexylmethan oder 3,3'-Dimethyl-4,4'-diamino-dicyclohexylmethan. Besonders bevorzugt sind 3,3'-Dimethyl-4,4'-diamino-dicyclohexylmethan, 1,5-Diaminopentan, 2,4'- und/oder 4,4'-Diaminodicyclohexylmethan, 1,5-Diamino-2-methylpentan und ganz besonders bevorzugt ist die Verwendung von 2,4'- und/oder 4,4'-Diaminodicyclohexylmethan.

Besonders bevorzugt sind Polyasparaginsaureester-haltige Komponenten A Zusammensetzungen enthaltend einen oder mehrere Polyasparaginsäureester der allgemeinen Formeln (I) und (II), bei denen X für organische Reste steht, die durch Entfernung der primären Aminogruppen aus einem der Polyamine der allgemeinen Formel (III) erhalten wird, ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan.

Ganz besonders bevorzugt sind Polyasparaginsaureester-haltige Komponenten A Zusammensetzungen enthaltend einen oder mehrere Polyasparaginsäureester der allgemeinen Formeln (I) und (II), bei denen X für organische Reste steht, die durch Entfernung der primären Aminogruppen aus einem der Polyamine der allgemeinen Formel (III) erhalten wird, ausgewählt aus der Gruppe: 3,3'-Dimethyl-4,4'diaminodicyclohexylmethan, 2,4'- und/oder 4,4'-Diaminodicyclohexylmethan, 1,5-Diamino-2-methylpentan.
Index m steht für eine ganze Zahl >1 und bevorzugt für 2.

Bevorzugt sind Polyasparaginsaureester-haltige Komponenten A Zusammensetzungen enthaltend einen oder mehrere Polyasparaginsäureester der allgemeinen Formeln (I) und (II), mit einem Anteil von 1% bis 20%, bevorzugt 4% bis 20%, besonders bevorzugt bis 4% bis 15% der GC Oberfläche (gemessen als Flächen-% im Gas Chromatogram) an Verbindungen der allgemeinen Formel (II), wobei die Summe der GC Oberflächen der Verbindungen der beiden allgemeinen Formeln (I) und (II) 100% beträgt.

Bevorzugt sind Polyasparaginsaureester-haltige Komponenten A Zusammensetzungen enthaltend einen oder mehrere Polyasparaginsäureester der allgemeinen Formeln (I) und Formel (II), mit einem Anteil von 0,01 bis 1,2 Gew.-%, bevorzugt 0,01 bis 1 Gew.-%, besonders bevorzugt 0,01 bis 0,1 Gew.-%, bezogen auf das Gesamtgewicht der Komponente A, an Fumarsäuredialkylester.

Besonders bevorzugt sind Polyasparaginsäureester-haltige Komponenten A Zusammensetzungen enthaltend einen oder mehrere Polyasparaginsäureester der allgemeinen Formel (I),
in welcher

| | |
|---|---|
| X | für einen m-wertigen, gegebenenfalls ein oder mehrere Heteroatome enthaltenden organischen Rest steht, wie er durch Entfernung von primären Aminogruppen aus Polyetherpolyaminen mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimethyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan erhalten wird, |
| R1 und R2 | für gleiche oder verschiedene Alkylreste mit je 1 bis 8 Kohlenstoffatomen stehen, |
| m | für eine ganze Zahl > 1 steht, |

und
einen oder mehrere Polyasparaginsäureester mit primärer Aminogruppe der allgemeinen Formel (II),
in welcher

| | |
|---|---|
| n | für m-1 steht, |
| X, | Reste R1 und R2 die oben genannten Bedeutungen haben, |

dadurch gekennzeichnet, dass der Anteil der Verbindungen der allgemeinen Formel (II) von 1% bis 20% der GC Oberfläche (gemessen als Flächen-% im Gas Chromatogram) entspricht, wobei die Summe der GC Oberflächen der Verbindungen der beiden allgemeinen Formeln (I) und (II) 100% beträgt und in Komponente A Fumarsäuredialkylester in Mengen von 0,01 bis 1,2 Gew.-%, bezogen auf das Gesamtgewicht der Komponente A, vorliegen.

Ganz besonders bevorzugt sind Polyasparaginsaureester-haltige Komponenten A Zusammensetzungen enthaltend einen oder mehrere Polyasparaginsäureester der allgemeinen Formel (I)
in welcher

| | |
|---|---|
| X | für einen m-wertigen organischen Rest steht, wie er durch Entfernung von primären Aminogruppen aus |

3,3'-Dimethyl-4,4'diaminodicyclohexylmethan, 2,4'- und/oder 4,4'-Diaminodicyclohexylmethan, 1,5-Diamino-2-methylpentan erhalten werden kann,

R1 und R2 für gleiche oder verschiedene Alkylreste ausgewählt aus der Gruppe Methyl-, Ethyl-, Propyl-, iso-Propyl-, Butyl- oder iso-butyl-Reste stehen,

m für 2 steht,

und
einen oder mehrere Polyasparaginsäureester mit primärer Aminogruppe der allgemeinen Formel (II),
in welcher

n für m-1 steht,

X, Reste R1 und R2 die oben genannten Bedeutungen haben,

dadurch gekennzeichnet, dass der Anteil der Verbindungen der allgemeinen Formel (II) von 4% bis 20% der GC Oberfläche (gemessen als Flächen-% im Gas Chromatogram) entspricht, wobei die Summe der GC Oberflächen der Verbindungen der beiden allgemeinen Formeln (I) und (II) 100% beträgt und in Komponente A Fumarsäuredialkylester in Mengen von 0,01 bis 1 Gew.-%, bezogen auf das Gesamtgewicht der Komponente A, vorliegen.

Noch mehr bevorzugt sind Polyasparaginsaureester-haltige Komponenten A Zusammensetzungen enthaltend einen oder mehrere Polyasparaginsäureester der allgemeinen Formel (I),
in welcher

X für einen m-wertigen organischen Rest steht, wie er durch Entfernung von primären Aminogruppen aus 3,3'-Dimethyl-4,4'diamino-dicyclohexylmethan, 2,4'- und/oder 4,4'-Diaminodicyclohexylmethan erhalten werden kann,

R1 und R2 für Ethyl-Reste stehen,

m für 2 steht,

und
einen oder mehrere Polyasparaginsäureester mit primärer Aminogruppe der allgemeinen Formel (II),
in welcher

n für m-1 steht,

X, Reste R1 und R2 die oben genannten Bedeutungen haben,

dadurch gekennzeichnet, dass der Anteil der Verbindungen der allgemeinen Formel (II) von 4% bis 15% der GC Oberfläche (gemessen als Flächen-% im Gas Chromatogram) entspricht, wobei die Summe der GC Oberflächen der Verbindungenen der beiden allgemeinen Formel (I) und (II) 100% beträgt und in Komponente A Fumarsäuredialkylester in Mengen von 0,01 bis 0,1 Gew.-%, bezogen auf das Gesamtgewicht der Komponente A, vorliegen.

Bevorzugt sind Polyasparaginsaureester-haltige Komponenten A Zusammensetzungen enthaltend einen oder mehrere Polyasparaginsäureester der allgemeinen Formel (I) und Formel (II), die eine Platin-Cobalt-Farbzahl ≤ 100, besonders bevorzugt ≤ 50 aufweisen. Die Messung der Platin-Cobalt-Farbzahl erfolgt gemäß DIN EN ISO 6271:2016-05.

Polyasparaginsaureester-haltige Komponenten A, wie zuvor beschrieben, lassen sich durch das folgende Verfahren herstellen:

Umsetzung von Polyaminen der allgemeinen Formel (III),

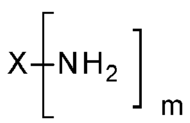

(III),

wobei X
für einen m-wertigen, gegebenenfalls ein oder mehrere Heteroatome enthaltenden organischen Rest steht, wie er durch Entfernung der primären Aminogruppen aus einem (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,

m für eine ganze Zahl > 1, bevorzugt für 2 steht,
mit Verbindungen der allgemeinen Formel (IV)

R1OOC-CH=CH-COOR2 (IV),

wobei R1 und R2
für gleiche oder verschiedene organische Reste, vorzugsweise für gleiche oder verschiedene Alkylreste mit je 1 bis 18 Kohlenstoffatomen, besonders bevorzugt für gleiche oder verschiedene Alkylreste mit je 1 bis 8 Kohlenstoffatomen, ganz besonders bevorzugt für jeweils Alkylreste wie Methyl-, Ethyl-, Propyl-, iso-Propyl-, Butyl- oder iso-Butyl-Reste und am meisten bevorzugt für Ethyl stehen,
und einer destillativen Entfernung des nicht umgesetzten Anteils an der Verbindung der allgemeinen Formel (IV),.

Vorzugsweise erfolgt das oben beschriebene Verfahren zur Herstellung der Polyasparaginsaureester-haltigen Komponenten A in zwei Schritten. Im ersten Schritt werden die Verbindungen der allgemeinen Formel (III) und (IV) bei Temperaturen zwischen 0°C und 100°C, vorzugsweise 20° bis 80°C und besonders bevorzugt 20° bis 60°C, in einem Verhältnis der Äquivalente der primären Aminogruppen der Verbindungen der allgemeinen Formel (III) zu den C=C-Doppelbindungsäquivalenten der Verbindungen der allgemeinen Formel (IV) von 1:1.2 bis 1.2:1, vorzugsweise jedoch 1:1.05 bis 1.05:1 bis zu einem Restgehalt an Verbindungen der allgemeinen Formel (IV) von 2 bis 15 Gew.-Prozent, bevorzugt von 3 bis 10 Gew.-Prozent umgesetzt.

Im zweiten Schritt wird der nicht umgesetzte Anteil an der Verbindungen der allgemeinen Formel (IV) destillativ entfernt.

Geeignete Bedingungen während der Destillation sind ein Druckbereich zwischen 0.01 und 2 mbar und eine Temperatur des Sumpfablaufs beim Austritt aus der Destillationsapparatur ≤ 170°C und ≥ der Temperatur liegt, die sich aus der folgenden Formel (V) ergibt:

$$T(\text{Sumpfablauf}) = 27 \times \ln(p) + 150 \quad (V)$$

wobei

| | |
|---|---|
| T(Sumpfablauf) | für die Temperatur des Sumpfablaufs in °C und |
| p | für den Druck in der Destillationsapparatur in mbar stehen. |

Durch Einhalten dieses Druckbereichs wird gewährleistet, dass einerseits moderate Temperaturen im Sumpfablauf ausreichen, um die Fumarsäuredialkylester im gewünschten Umfang abzureichern, andererseits das Verfahren im technischen Maßstab anwendbar bleibt. Bei geringerem Druck wird die Gasdichte zu gering und die erforderlichen Apparate deshalb so groß, dass das Verfahren aus wirtschaftlicher Sicht nachteilig wird.

Bevorzugt ist die Temperatur des Sumpfablaufs ≤ 170°C, dabei jedoch mindestens 20 K oberhalb der Temperatur, die sich aus der Formel (V) ergibt, besonders bevorzugt liegt sie zwischen 20 K und 40 K oberhalb der Temperatur, die sich aus der Formel (V) ergibt jedoch nicht oberhalb von 170°C.

Verbindungen der allgemeinen Formel (III), die in dem oben beschriebenen Verfahren Anwendung finden sind alle bekannten Polyamine mit primären Aminogruppen, die der allgemeinen Formel (III) entsprechen. Beispielhaft seien die folgenden Verbindungen genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (Dytek®A, Fa DuPont), 1,6-Diaminohexan, 2,2,4- und/oder 2,4,4-Trimethyl-1,6-diaminohexan, 1,11-Diaminoundecan,1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, amino-funktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Bevorzugt werden im oben beschriebenen Verfahren Polyamine der allgemeinen Formel (III) eingesetzt worin m = 2 bedeutet und X für einen cyclischen Kohlenwasserstoffrest mit mindestens einem cyclischen Kohlenstoffring steht. Beispiele für besonders bevorzugt einsetzbare Diamine sind 1-Amino-3,3,5-trimethyl-5-aminomethylcyclohexan (IPDA), 2,4- und/oder 2,6-Hexahydrotoluylendiamin (H6-TDA), Isopropyl-2,4-diaminocyclohexan, und/oder Isopropyl-2,6-diaminocyclohexan, 1,3-Bis-(aminomethyl)-cyclohexan, 2,4'- und/oder 4,4'-Diaminodicyclohexylmethan, 3,3'-Dimethyl-4,4'-diamino-dicyclohexylmethan (Laromin® C 260, Fa. BASF AG), die isomere, eine Methylgruppe als Kernsubstituenten aufweisende Diaminodicyclohexylmethane (=C-Monomethyl-diaminodicyclohexylmethane), 3(4)-Aminomethyl-l-methylcyclohexylamin (AMCA) sowie araliphatische Diamine, wie z.B. 1,3-Bis-(aminomethyl)-benzol oder m-Xylylendiamin.

Ebenfalls bevorzugt werden im erfindungsgemäßen Verfahren Polyamine der allgemeinen Formel (III) eingesetzt, ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'-diamino-dicyclohexylmethan. Besonders bevorzugt sind 3,3'-Dimethyl-4,4'-diamino-dicyclohexylmethan, 2,4'- und/oder 4,4'-Diaminodicyclohexylmethan, 1,5-Diamino-2-methylpentan und ganz besonders bevorzugt ist die Verwendung von 2,4- und/oder 4,4'-Diaminodicyclohexylmethan.

Besonders bevorzugt werden im erfindungsgemäßen Verfahren Polyamine der allgemeinen Formel (III) eingesetzt, ausgewählt aus der Gruppe: Polyetherpolyamine mit aliphatisch gebundenen primären Aminogruppen, 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaininohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan.

Ganz besonders bevorzugt werden im erfindungsgemäßen Verfahren Polyamine der allgemeinen Formel (III) eingesetzt, ausgewählt aus der Gruppe 3,3'-Dimethyl-4,4'diaminodicyclohexylmethan, 2,4'- und/oder 4,4'-Diaminodicyclohexylmethan, 1,5-Diamino-2-methylpentan.

Bevorzugte Verbindungen der allgemeinen Formel (IV), die in dem oben beschriebenen Verfahren Anwendung finden sind Malein- oder Fumarsäureester der allgemeinen Formel (IV), worin R1 und R2 für gleiche oder verschiedene organische Reste mit je 1 bis 18 Kohlenstoffatomen stehen. Bevorzugt stehen R1 und R2 unabhängig voneinander für lineare oder verzweigte Alkylreste mit 1 bis 8 Kohlenstoffatomen, besonders bevorzugt für jeweils Alkylreste wie Methyl-, Ethyl-, Propyl-, iso-Propyl-, Butyl- oder iso-Butyl-Reste und ganz besonders bevorzugt für Ethyl.
Beispielhaft für Verbindungen der allgemeinen Formel (IV) seien die folgenden Verbindungen genannt: Maleinsäuredimethylester, -diethylester, -di-n oder -iso-propylester, -di-n-butylester, -di-2-ethyl-hexylester oder die entsprechenden Fumarsäureester. Ganz besonders bevorzugt ist Maleinsäurediethylester.

Die erfindungsgemäßen zweikomponentigen Beschichtungszusammensetzungen enthalten mindestens eine Polyisocyanatkomponente B .
Geeignete Polyisocyanatkomponenten B sind organische Polyisocyanate mit einer mittleren NCO-Funktionalität von mindestens 2 und einem Molekulargewicht von mindestens 140 g/mol. Gut geeignet sind vor allem unmodifizierte organische Polyisocyanate des Molekulargewichtsbereichs 140 bis 300 g/mol, Lackpolyisocyanate eines Molekulargewichts im Bereich von 300 bis 1000 g/mol, sowie Urethan-, Harnstoff- und / oder Allophanatgruppen- aufweisende NCO-Prepolymere eines über 400 g/mol liegenden Molekulargewichtes oder deren Gemische.
Unter dem Begriff "Lackpolyisocyanate" sind im Sinne der Erfindung Verbindungen oder Gemische von Verbindungen zu verstehen, die durch an sich bekannte Oligomerisierungsreaktion von einfachen Polyisocyanaten erhalten werden können. Geeignete Oligomerisierungsreaktionen sind z.B. die Carbodiimidisierung, Dimerisierung, Trimerisisierung, Biuretisierung, Harnstoffbildung, Urethanisierung, Allophanatisierung und/oder Cyclisierung unter Ausbildung von Oxadiazinstrukturen. Bei Oligomerisierung können mehrere der genannten Reaktionen gleichzeitig oder nacheinander ablaufen.
Bevorzugt handelt es sich bei den "Lackpolyisocyanaten" um Biuretpolyisocyanate, Isocyanuratgrupen-aufweisende Polyisocyanate, Isocyanurat- und Uretdiongruppen-aufweisende Polyisocyanatgemische, Urethan- und/oder Allophanatgruppen-aufweisende Polyisocyanate oder um Isocyanurat- und Allophanatgruppen-aufweisende Polyisocyanatgemische auf Basis einfacher organischer Polyisocyanate.
Ebenfalls geeignet als Polyisocyanatkomponente B sind die an sich bekannten Isocyanatgruppen aufweisenden Prepolymere auf Basis einfacher organischer Polyisocyanate und/oder auf Basis von Lackpolyisocyanaten einerseits und organischen Polyhydroxyverbindungen eines über 300 g/mol liegenden Molekulargewichts andererseits. Während es sich bei den Urethangruppen-aufweisenden Lackpolyisocyanaten um Derivate von niedermolekularen Polyolen des Molekulargewichtsbereichs von 62 bis 300 g/mol handelt, geeignete Polyole sind beispielsweise Ethylenglykol, Propylenglykol, Trimethylolpropan, Glycerin oder Gemische dieser Alkohole, werden zur Herstellung der Isocyanatgruppen aufweisenden Prepolymeren Polyhydroxyverbindungen eines über 300 g/mol, bevorzugt über 400 g/mol, besonders bevorzugt eines zwischen 400 und 8000 g/mol liegenden Molekulargewichts eingesetzt. Derartige Polyhydroxylverbindungen sind insbesondere solche, die pro Molekül 2 bis 6, bevorzugt 2 bis 3 Hydroxylgruppen aufweisen und aus der Gruppe, bestehend aus Ether-, Ester-, Thioether-, Carbonat- und Polyacrylatpoloyolen und Gemischen aus derartigen Polyolen ausgewählt sind.
Bei der Herstellung der Isocyanatgruppen aufweisenden Prepolymeren können die genannten höhermolekularen Polyole auch in Abmischungen mit den genannten niedermolekularen Polyolen zur Anwendung gelangen, so dass unmittelbar Gemische aus niedermolekularen, Urethangruppen aufweisenden Lackpolyisocyanaten und höhermolekularen NCO-Prepolymeren resultieren, die ebenfalls als erfindungsgemäße Polyisocyanatkomponente b) geeignet sind.
Zur Herstellung der Isocyanatgruppen aufweisenden Prepolymeren oder deren Gemischen mit den Lackpolyisocyanaten

werden einfache organische Polyisocyanate der nachstehend beispielhaft genannten Art oder Lackpolyisocynate mit den höhermolekularen Hydroxylverbindungen oder deren Gemischen mit niedermolekularen Polyhydroxylverbindungen der beispielhaft genannten Art unter Einhaltung eines NCO/OH Aquivalentverhaltnisses von 1.1:1 bis 40:1, bevorzugt 2:1 bis 25:1 unter Urethan und / oder Allophanatbildung umgesetzt. Gegebenenfalls kann bei Verwendung eines Überschusses an destillierbarem einfachem organischem Polyisocyanat dieser im Anschluss an die Umsetzung destillativ entfernt werden, so dass monomerenfreie Isocyanatgruppen aufweisende NCO-Prepolymere vorliegen, die ebenfalls als Polyisocyanatkomponente b) eingesetzt werden können.
Beispiele für geeignete einfache organische Polyisocyanate sind 1,4-Diisocyanatobutan, 1,5-Diisocyanatopentan, 1,6-Diisocyanatohexan (HDI), 1,5-Diisocyanato-2,2-dimethylpentan, 2,2,4-bzw. 2,4,4-Trimethyl-1,6-diisocyanatohexan, Tetramethylxylylendiisocyanat (TMXDI) 1-Isocyanato-3,3,5-trimethyl-5-isocyanatomethylcyclohexan (Isophorondiisocyanat, IPDI), 1-Isocyanato-1-methyl-4-(3)-isocyanatomethylcyclohexan, Dicyclohexylmethan-2,4'-und/oder 4,4'-diisocyanat, 1,10-Diisocyanatodecan, 1,12-Diisocyanatododecan, Cyclohexan-1,3- und -1,4-diisocyanat, Xylylendiisocyanat-Isomere, Triisocyanatononan (TIN), Naphthylen-1,5-diisocyanat, 2,4-Diisocyanatoluol oder dessen Gemische mit 2,6-Diisocyanatotoluol mit bevorzugt, bezogen auf Gemische, bis zu 35 Gew.-% 2,6-Diisocyanatotoluol, 2,2'-, 2,4'-, 4,4'-, Diisocyanatodiphenylmethan oder technische Polyisocyanatgemische der Diphenylmethanreihe, oder beliebige Gemische der genannten Polyisocyanate.
Bevorzugt kommen dabei aliphatische, cycloaliphatische oder araliphatische Polyisocyanate, ausgewählt aus der Gruppe 1,4-Diisocyanatobutan, 1,5-Diisocyanatopentan, 1,6-Diisocyanatohexan (HDI), 1,5-Diisocyanato-2,2-dimethylpentan, 2,2,4- bzw. 2,4,4-Trimethyl-1,6-diisocyanatohexan, Tetramethylxylylendiisocyanat (TMXDI) 1-Isocyanato-3,3,5-trimethyl-5-isocyanatomethylcyclohexan (Isophorondiisocyanat, IPDI), 1-Isocyanato-1-methyl-4-(3)-isocyanatomethylcyclohexan, Dicyclohexylmethan-2,4'-und/oder 4,4'-diisocyanat, 1,10-Diisocyanatodecan, 1,12-Diisocyanatododecan, Cyclohexan-1,3- und -1,4-diisocyanat, Xylylendiisocyanat-Isomere, Triisocyanatononan (TIN), oder beliebige Gemische derartiger Polyisocyanate, zum Einsatz.

Prinzipiell ist natürlich auch der Einsatz von Mischungen verschiedener Polyisocyanatkomponenten der vorstehend genannten Art möglich.

Neben der Polyasparaginsaureester-haltigen Komponente A kann die erfindungsgemäße zweikomponentige Zusammensetzung weitere gegenüber Isocyanatgruppen reaktionsfähige Komponenten (Komponenten C) enthalten. Dies können beispielsweise niedermolekulare Polyole des Molekulargewichtsbereichs von 62 bis 300 g/mol, beispielsweise Ethylenglykol, Propylenglykol, Trimethylolpropan, Glycerin oder Gemische dieser Alkohole, oder Polyhydroxyverbindungen eines über 300 g/mol, bevorzugt über 400 g/mol, besonders bevorzugt eines zwischen 400 und 20000 g/mol liegenden Molekulargewichts sein. Derartige Polyhydroxylverbindungen sind insbesondere solche, die pro Molekül 2 bis 6, bevorzugt 2 bis 3 Hydroxylgruppen aufweisen und aus der Gruppe, bestehend aus Ether-, Ester-, Thioether-, Polyurethan-, Carbonat- und Polyacrylatpoloyolen und Gemischen aus derartigen Polyolen ausgewählt sind.

Weiterhin enthält die erfindungsgemäße zweikomponentige Zusammensetzung mindestens einen Wasserfänger und/oder ein Hydrolyseschutzmittel (Komponente D1).
Als Wasserfänger kommen z.B. Triethylorthoformiat, Toluolsulfoisocyanat, Monooxazolidine oder Molekularsiebe, als Hydrolyseschutzmittel z.B. Carbodiimide in Betracht.

Darüberhinaus kann die erfindungsgemäße Zusammensetzung weitere, für die Beschichtungstechnologie mit Polyisocyanat-Polyadditionsverbindungen, insbesondere für Polyurethan verbindungen typische Hilfs- und Zusatzstoffe enthalten (Komponente D2). Beispiele sind Katalysatoren/Aktivatoren wie zum Beispiel Titan-, Zirkonium-, Bismut-, Zinn- und/oder eisenhaltigen Katalysatoren, wie sie beispielsweise in WO 05058996 beschrieben sind. Möglich ist auch eine Zugabe von Aminen oder Amidinen.
Beispiele weiterer geeigneter Hilfs- und Zusatzstoffe D2 sind insbesondere Lichtschutzmittel wie UV-Absorber und sterisch gehinderte Amine (HALS), weiterhin Stabilisatoren, Entschäumungs-, Antikrater- und/oder Netzmittel, Verlaufsmittel, filmbildende Hilfsmittel, Reaktivverdünner, Biozide, Lösemittel, oder Substanzen zur Rheologiesteuerung,
Der Einsatz von Lichtschutzmitteln insbesondere von UV-Absorber wie beispielsweise substituierten Benztriazolen, S-Phenyltriazinen oder Oxalaniliden sowie sterisch gehinderten Aminen insbesondere mit 2,2,6,6-Tetramethyl-piperidyl-Strukturen - als HALS bezeichnet - ist beispielhaft beschrieben in A. Valet, Lichtschutzmittel für Lacke, Vincentz Verlag, Hannover, 1996.
Stabilisatoren wie beispielsweise Radikalfänger und andere Polymerisationsinhibitoren wie sterisch gehinderte Phenole stabilisieren Lackkomponenten während der Lagerung und sollen Verfärbungen während der Aushärtung verhindern. Netz- und Verlaufsmittel verbessern die Oberflächenbenetzung und/oder den Verlauf von Lacken. Beispiele sind Fluortenside, Silikontenside sowie spezielle Polyacrylate. Rheologiesteuernde Additive sind wichtig um die Eigenschaften des Zwei-Komponenten-Systems bei der Applikation und in der Verlaufsphase auf dem Substrat zu steuern und sind beispielsweise aus den Patentschriften WO 9422968, EP0276501, EP0249201 oder WO 9712945 bekannt.
Die Zusammensetzung kann Lösungsmittel enthalten. Das Lösungsmittel kann ein organisches Lösungsmittel oder ein Gemisch organischer Lösungsmittel sein oder Wasser oder ein Gemisch aus organischen Lösungsmittel(n) und Wasser. Geeignete Lösungsmittel sind in dem Fachmann bekannter Art und Weise abgestimmt auf die Zusammensetzung sowie

das Applikationsverfahren einzusetzen. Lösemittel sollen die eingesetzten Komponenten lösen und deren Vermischung fördern sowie Unverträglichkeiten vermeiden. Weiterhin sollen sie während der Applikation und der Härtung die Beschichtung auf die ablaufende Vernetzungsreaktion abgestimmt verlassen, so dass eine lösemittelfreie Beschichtung mit möglichst guter Optik und ohne Fehlstellen wie Kocher oder Nadelstiche entsteht. Insbesondere kommen Lösemittel in Betracht, die in der Zwei Komponenten Technologie zur Anwendung kommen. Beispiele für organische Lösungsmittel sind Ketone wie Aceton, Methylethylketon oder Hexanon, Ester wie Ethylacetat, Butylacetat, Methoxyproylacetat, substituierte Glykole und andere Ether, Aromaten wie Xylol oder Solventnaphtha wie z.B. der Fa. Exxon-Chemie sowie Gemische der genannten Lösemittel. Liegt der NCO-reaktive Teil der Zusammensetzung als wässrige Dispersion vor, eignet sich auch Wasser als Löse- bzw. Verdünnungsmittel.

Vorzugsweise beträgt das Verhältnis der Polyisocyanatkomponente B zu der Polyasparaginsaureester-haltigen Komponente A in der Zusammensetzung bezogen auf die Stoffmengen der Polyisocyanatgruppen zu den NCO-reaktiven Gruppen von 0,5 zu 1,0 bis 3,0 zu 1,0. Besonders bevorzugt ist ein Verhältnis von 0,9 zu 1,0 bis 1,5 zu 1,0. Ganz besonders bevorzugt ist ein Verhältnis von 1,05 zu 1,0 bis 1,25 zu 1,0.

Die erfindungsgemäße zweikomponentige Zusammensetzung ist vorzugsweise keine aufschäumbare oder schaumbildende Zusammensetzung. Die Zusammensetzung ist vorzugsweise nicht radikalisch polymerisierbar, insbesondere nicht photo-polymerisierbar, d.h. die Zusammensetzung härtet nicht durch radikalische Prozesse aus, insbesondere nicht durch radikalische Polymerisationsprozesse die durch aktinische Strahlung initiiert werden.

Die Herstellung der erfindungsgemäßen zweikomponentigen Beschichtungszusammensetzung erfolgt nach in der Technologie der Lacke an sich bekannten Methoden.

Eine isocyanatreaktive (R)und eine isocyanathaltige Komponente (H) werden zunächst getrennt durch Vermischen der jeweiligen isocyanatreaktiven Komponenten A und C, bzw. durch Vermischen der jeweiligen Polyisocyanatkomponenten B hergestellt. Die Hilfs- und Zusatzstoffe D1 und D2 werden bevorzugt der isocyanatreaktiven Komponente R beigemischt. Ein Vermischen der so hergestellten Komponenten R und H erfolgt erst unmittelbar vor oder während der Applikation. Erfolgt das Vermischen vor der Applikation ist zu beachten, dass die Reaktion der Bestandteile nach dem Vermischen bereits startet. Je nach Auswahl der Komponenten und der Zusatzstoffe wird die Reaktion unterschiedlich schnell ablaufen. Die Verarbeitungszeit innerhalb derer die Zusammensetzung appliziert werden muss, auch als Topfzeit bezeichnet und definiert als die Zeit von der Vermischung der Komponenten bis zur Verdoppelung der Auslaufzeit, liegt dann je nach Auswahl der Komponenten im Bereich von 1 Minute bis zu 24 Stunden, meist im Bereich von 10 Minuten bis 8 Stunden. Die Bestimmung der Topfzeit erfolgt nach dem Fachmann bekannten Methoden.

Die Erfindung betrifft auch ein Verfahren zum Herstellen einer Beschichtung auf einem Substrat, welches mindestens folgende Schritte umfasst:

i) Aufbringen der vorstehend beschriebenen zweikomponentigen Beschichtungszusammensetzung auf mindestens einen Teil eines zu beschichtenden Substrats, und

ii) Härten der Beschichtungszusammensetzung aus Schritt i).

Weitere Gegenstände der vorliegenden Erfindung sind daher die Verwendung der erfindungsgemäßen zweikomponentigen Beschichtungszusammensetzungen zur Herstellung von Beschichtungen auf Substraten, das oben beschriebene Verfahren zum Beschichten eines Substrats, sowie die so erhältlichen beschichteten Substrate selbst.

Die Substrate können bereits mit einer oder mehreren Lackschichten ganz oder teilweise beschichtet sein. Diese Lackschichten können noch ungehärtet oder feucht, teilgehärtet oder vollständig gehärtet sein, vorzugsweise sind die weiteren Lackschichten auf dem Substrat teilgehärtet oder vollständig gehärtet. Beispiele für Lackschichten sind Grundierungen, Primer, Füller, Spachtel, Basislacke oder bereits vollständig lackierte Substrate, die nach einer eventuellen Vorbehandlung wie Anschleifen oder Plasmaaktivierung erneut überschichtet werden. Insbesondere sind Substrate, wie sie in der Reparaturlackierung oder Lackierung im Rahmen von Renovierungen oder Wartungen z.B. bei Fahrzeugen, insbesondere bei Schiffen, Flugzeugen, Kraftfahrzeugen wie Autos, Lastkraftwagen, Bussen, Großfahrzeugen, Bahnfahrzeugen auftreten, geeignet.

Weitere, bevorzugte Gegenstände der vorliegenden Erfindung sind daher die Verwendung der vorstehend beschriebenen zweikomponentigen Beschichtungszusammensetzungen zur Herstellung von Klarlacken auf Substraten, insbesondere solchen, wie sie in der Reparaturlackierung oder Lackierung im Rahmen von Renovierungen oder Wartungen, insbesondere bei Fahrzeugen auftreten, das oben beschriebene Verfahren zum Beschichten dieser speziellen Substrate, sowie die so erhältlichen beschichteten Substrate selbst.

Die Beschichtungszusammensetzung kann durch herkömmliche Auftragungsverfahren aufgetragen werden. Beispiele für Auftragsverfahren sind Bürsten, Walzenauftrag, Rakeln, Tauchen und Sprühen, bevorzugt ist ein Sprühauftrag. Nach einer optionalen Ablüftzeit An das Aufbringen schließt sich die Aushärtung bzw. die Trocknung der erfindungsgemäßen Zusammensetzung auf dem Substrat bzw. Objekt an. Diese erfolgt nach den in der Beschichtungstechnologie üblichen Methoden entweder unter Umgebungsbedingungen bezüglich Temperatur und Luftfeuchte oder unter forcierten Bedingungen beispielsweise durch Temperaturerhöhung in Öfen, Einsatz von Strahlung wie beispielsweise

Infrarot- oder Nahinfrarot- oder Mikrowellenstrahlung, sowie Einsatz von entfeuchteter und/oder erwärmter Luft oder anderen Gasen. Bevorzugt ist dabei, auf den Einsatz von Geräten zur forcierten Aushärtung zu verzichten. Die aufgetragene Beschichtungszusammensetzung wird zum Beispiel bei Temperaturen von -20 bis 100°C, vorzugsweise von -10 bis 80°C, bevorzugter von 0 bis 60°C und am meisten bevorzugt von 10 bis 40°C ausgehärtet. Auch wenn nicht bevorzugt, geringere Härtungstemperaturen können ebenfalls verwendet werden, werden jedoch zu längeren Härtungszeiten führen.
Es ist ebenfalls möglich, auch wenn nicht bevorzugt, die Zusammensetzung bei höheren Temperaturen, beispielsweise 80 bis 160°C oder höher, auszuhärten.
Nach dem Aushärten der ersten Überzugsschicht kann eine weitere Überzugsschicht aufgebracht werden und ebenfalls ausgehärtet.

Ein weiterer Gegenstand der Erfindung ist die Verwendung der erfindungsgemäßen zweikomponentigen Beschichtungszusammensetzung zur Herstellung von wasserbeständigen Beschichtungen.

## Experimenteller Teil

### Rohstoffe und Substrate:

PACM 20: ein Gemisch aus 2,4 und 4,4'-Diaminodicyclohexylmethan, Hersteller Fa. Evonik Desmodur N 3900: ein niedrigviskose HDI-Trimerisat mit ca. 23,5% NCO, Hersteller Fa. Covestro Byk 331: Polyether modifiziert Polydimethylsiloxan Oberflächenadditiv, Hersteller Fa. BYK Tinuvin 384-2: Benzenpropansäure, 3-(2H-benztriazol-2-yl)-5-(1,1-dimethylethyl)-4-hydroxy-, C7-9-verzweigt und linear Alkylester, ein Lichtstabilisator von der Fa. BASF
Additive OF: Triethylorthoformiat, ein Additiv von der Fa. Borchers
Substrat: Coil Coat beschichtete Prüftafeln, grau, Lieferant: Fa. Heinz Zanders Prüf-Blech-Logistik in Solingen

### Methoden:

Die Fumarsäurediethylester-Gehalte wurden quantitativ mit Hilfe einer GC-Methode mit einem internen Standard bestimmt. Es wurde ein 6890 Gaschromatograph der Fa. Agilent mit einer Standard-GC-Kapillare (100% Polysiloxan-Phase) und einem FID Detektor verwendet. Die Temperatur des Injektors (Splitausgang) war 180°C, als Trägergas wurde Helium verwendet. Die Bestimmungsgrenze diese Methode betrug 300 ppm.

GC-MS Messungen wurden mit einem 6890 Gaschromatograph und Massen Spektrum Detektor 5973 der Fa. Agilent mit Standard-Ionisierung (electron impact) mit 70eV, eine Standard-GC-Kapillare (100% Polysiloxan-Phase) und Split-Injektion bei 250°C Injektortemperatur durchgeführt. Ausgewertet wurden die % Flächen des Gaschromatograms.

Sämtliche Viskositatsmessungen erfolgten mit einem Physica MCR 51 Rheometer der Fa. Anton Paar Germany GmbH (DE) nach DIN EN ISO 3219:1994-10.

Die Hazen-Farbzahlen wurden an einem LICO 400 Farbmessgerät der Fa. Hach Lange GmbH, Düsseldorf, gemäß DIN EN ISO 6271:2016-05 ermittelt.

Die Bestimmung der Aminzahlen erfolgte titrimetrisch nach EN ISO 9702:1998 (Perchlorsäre-Methode) mit der Ausnahme, dass die Ergebnisse als Aminzahl angegeben wurden. Die Aminzahl in mg KOH/g wurde nach folgender Gleichung berechnet:

$$Aminzahl = \frac{(a-b)x\,5{,}61}{E}$$

a: der Verbrauch, in Milliliter, an Perchlorsäure, c = 0,1 mol/l, im Hauptversuch;

b: der Verbrauch, in Milliliter, an Perchlorsäure, c = 0,1 mol/l, im Blindversuch;

E: die Einwaage, in Gramm.

Der Kondenswassertest wurde nach DIN EN ISO 6270-2 : 2017 durchgeführt. Die Bewertung der Blasenbildung erfolgte nach DIN EN ISO 4628-2 : 2016 (k.V. = keine Veränderung / Bl. = Blasenmenge / Gr. = Blasengröße)

### Beispiel 1 (Vergleich)

Kommerziell erhältlicher Polyasparaginsäureester der Fa. Covestro unter dem Namen Desmophen NH 1420.

Kenndaten:

| | |
|---|---|
| Monoamine der Formel (II) (GC-MS): | 4.0% |
| Fumarsäurediethylester (GC) | 2.9 Gew.-Prozent |
| Viskosität | 1220 mPas |
| Farbzahl | 27 APHA |
| Aminzahl | 201 mg KOH/g |

**Beispiel 2**

341.8g PACM 20 wurden bei 23 °C unter trockenem Stickstoff und Rühren vorgelegt. Dazu wurden 567,6g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C 2 Stunden gerührt. Anschließend wurde der Ansatz bei 23°C 7 Wochen gelagert. Der Gehalt an Fumarsäurediethylester betrug nach der Lagerung 2.7 Gew.-Prozent. Fumarsäurediethylester wurde dann bei 120 °C und 0.2 mbar abdestilliert. Man erhielt ein farbhelles Produkt mit folgenden Kenndaten:

| | |
|---|---|
| Monoamine der Formel (II) (GC-MS): | 5.3% |
| Fumarsäurediethylester (GC) | 0.08 Gew.-Prozent |
| Viskosität | 1810 mPas |
| Farbzahl | 19 APHA |
| Aminzahl | 203 mg KOH/g |

**Prüfung in Lacken**

Zubereitung eines Stammlackes

Zu der in der Tabelle 1 gegebenen Menge der Komponente A wurden die Additive sowie die in der Tabelle gegebene Menge Butylacetat gegeben und homogen verrührt.

Zubereitung der Härterlösung

Zu der in der Tabelle 1 gegebenen Menge der Komponente B wurde die gegebene Menge Butylacetat gegeben und homogen verrührt.

Beschichtungsaufbau: das Substrat wurde mit einem Basecoat Permahyd Serie 280, tiefschwarz, (Lieferant: Spies Hecker GmbH in Köln) mittels Luftdruckspritzverfahren mit einer Sata Jet RP, Düse 1,3 mm / 2,1 bar) beschichtet und 20 Minuten bei Raumtemperatur getrocknet, (Trockenfilmschichtstärke 15 µm). 2K-Klarlacke wurden hergestellt durch Vermischung von Komponente A und B unmittelbar vor Applikation durch Verrühren für 15 sec mit einem Holzspatel und im Luftdruckspritzverfahren mit einer Sata Jet RP, Düse 1,3 mm / 2,1 bar auf das vorbeschichtete Substrat angebracht. Die Trocknung erfolgte bei Raumtemperatur (24° C / 30 % rel. Luftfeuchte) über 7 Tage. Es wurden brillante, hochglänzende Beschichtungen mit einer Schichtdicke von 50-60 µm erhalten.

Tabelle 1. Zusammensetzung der Klarlacke

| | Beispiel 3 (Vergleich) | Beispiel 4 (Erfindungsgemäß) |
|---|---|---|
| **Komponente A** | | |
| Desmophen NH 1420 aus Beispiel 1 | 42,94 | |
| Polyasparaginsäureester aus Beispiel 2 | | 42,94 |
| Byk 331 (10%ig in BA) | 0,07 | 0,07 |
| Tinuvin 384-2 (50%ig in BA) | 3,53 | 3,53 |
| Additive OF (98%ig) | 3,00 | 3,00 |
| Butylacetat | 12,58 | 12,58 |

(fortgesetzt)

| **Komponente B** | | |
|---|---|---|
| Desmodur N 3900 (100%ig) | 27,89 | 28,44 |
| Butylacetat | 9,99 | 9,44 |
| Summe | 100,00 | 100,00 |

Tabelle 2. Ergebnisse des Kondenswassertests

| | Beispiel 3 (Vergleich) | Beispiel 4 (Erfindungsgemäß) |
|---|---|---|
| Schichtdicke µm | 51 | 63 |
| Glanz 20°/ Haze 0-Wert | 87/16 | 87 / 12 |
| | | |
| Kondenswassertest nach 24h | k.V. | k.V. |
| Kondenswassertest nach 48h | k.V. | k.V. |
| Kondenswassertest nach 72h | k.V. | k.V. |
| Kondenswassertest nach 96h | 2 Bl. Gr.2 | k.V. |
| Kondenswassertest nach 168h | 1 Bl. Gr.2, 1 Bl. Gr.5 | k.V. |
| Kondenswassertest nach 192h | 1 Bl. Gr.2, 1 Bl. Gr.5 | k.V. |
| Kondenswassertest nach 216h | 1 Bl. Gr.2,1 Bl. Gr.5 | k.V. |
| Kondenswassertest nach 240h | 1 Bl. Gr. 2, 1 Bl. Gr. 5, 2 Risse | 2 Bl. Gr. 2 |

Die Vergleich - Beschichtung aus dem Beispiel 3 zeigte schon nach 96h im Kondenswassertest Blasen, währen die erfindungsgemäße Beschichtung aus dem Beispiel 4 erst nach 240h eine erste Blasenbildung aufweist. Diese Ergebnisse erbringen den Nachweis, dass mit den erfindungsgemäßen Polyasparaginsäureester - Zusammensetzungen sich signifikante technische Vorteile gegenüber den herkömmlichen Beschichtungen erzielen lassen.

**Tabelle 3. Lacktechnische Eigenschaften von Beschichtungen**

| Temperatur: 24°C | | Beispiel 4 (erfindungsgemäß) | Beispiel 3 (Vergleich) |
|---|---|---|---|
| Luftfeuchte: 48% | | | |
| Festkörper in % bei Spritzviskosität (ber.) | | 74.9 | 81.1 |
| Auslaufzeit DIN 4mm (sec.) | 0h | 16 | 17 |
| | 5’ | 17 | 18 |
| | 10’ | 18 | 20 |
| | 15’ | 19 | 24 |
| | 20’ | 20 | 27 |
| | 25’ | 22 | 31 |
| | 30’ | 25 | 40 |
| Trocknung (min.) | T 1 | 12 | 15 |
| RT | T 3 | 25 | 30 |
| | T 4 | 40 | 45 |
| Schichtdicke (µm) | | ca. 50 | ca. 50 |
| Trocknung (min.) | T 1 | sofort | sofort |
| 30’ - 60°C | T 3 | 15 | 20 |
| | T 4 | 25 | 30 |
| Schichtdicke (µm) | | ca. 50 | ca. 50 |

Durch den Vergleich der in Tabelle 3 angegebenen lacktechnischen Eigenschaften von der Vergleich - Beschichtung (Beispiel 4) mit der erfindungsgemäßen Beschichtung (Beispiele 3)lässt sich eine schnellere Trocknung bei gleichbleibender Topfzeit der erfindungsgemäßen Beschichtungen erkennen.

## Patentansprüche

1. Zweikomponentige Beschichtungszusammensetzungen (2K-Beschichtungsmittel), enthaltend

    a) mindestens eine Polyasparaginsaureester-haltige Komponente A,
    b) mindestens eine Polyisocyanatkomponente B,
    c) gegebenenfalls eine oder mehrere, von A verschiedene, gegenüber Isocyanatgruppen reaktionsfähige Komponenten C und
    d) mindestens ein Wasserfänger und/oder Hydrolyseschutzmittel (Komponente D1) und gegebenenfalls weitere Hilfs- und Zusatzstoffe (Komponente D2),

    wobei Komponente A Zusammensetzungen entspricht, enthaltend
    einen oder mehrere Polyasparaginsäureester der allgemeinen Formel (I)

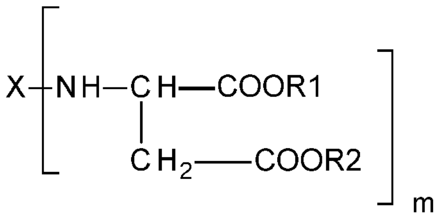

(I),

    in welcher

    X für einen m-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann,
    R1 und R2 für gleiche oder verschiedene organische Reste, mit je 1 bis 18 Kohlenstoffatomen stehen,
    m für eine ganze Zahl >1 steht,

    und einen oder mehrere Polyasparaginsäureester mit primärer Aminogruppe der allgemeinen Formel (II)

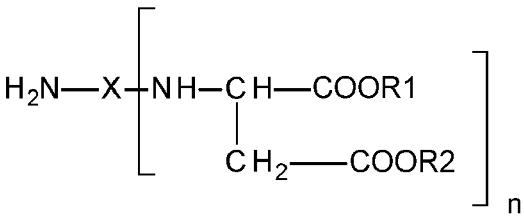

(II)

    in welcher

    n für m-1 steht,
    X, Reste R1 und R2 die oben genannten Bedeutungen haben,

    **dadurch gekennzeichnet, dass** der Anteil der Verbindungen der allgemeinen Formel (II) von 1% bis 20% der GC Oberfläche (gemessen als Flächen-% im Gas Chromatogram) entspricht, wobei die Summe der GC Oberflächen der Verbindungen der beiden allgemeinen Formeln (I) und (II) 100% beträgt und in Komponente A Fumarsäuredialkylester in Mengen von 0.01 bis 1,2 Gew.-%, bezogen auf das Gesamtgewicht der Komponente A, vorliegen.

2. Zweikomponentige Beschichtungszusammensetzungen (2K-Beschichtungsmittel) Anspruch 1, wobei der Anteil der Verbindungen der allgemeinen Formel II mindestens 4 % der GC Oberfläche (gemessen als Flächen-% im Gas Chromatogram) beträgt.

3. Zweikomponentige Beschichtungszusammensetzungen (2K-Beschichtungsmittel) gemäß Anspruch 1 oder 2, wobei der Anteil der Verbindungen der allgemeinen Formel II maximal 15 % der GC Oberfläche (gemessen als Flächen-% im Gas Chromatogram) beträgt.

4. Zweikomponentige Beschichtungszusammensetzungen (2K-Beschichtungsmittel) gemäß einem der Ansprüche 1 bis 3, **dadurch gekennzeichnet, dass** in Komponente A Fumarsäuredialkylester in einer Menge von 0,01 bis 1 Gew.-%, bezogen auf das Gesamtgewicht der Komponente A, vorliegen.

5. Zweikomponentige Beschichtungszusammensetzungen (2K-Beschichtungsmittel) gemäß einem der Ansprüche 1 bis 3, **dadurch gekennzeichnet, dass** in Komponente A Fumarsäuredialkylester in einer Menge von 0,01 bis 0,1 Gew.-%, bezogen auf das Gesamtgewicht der Komponente A, vorliegen.

6. Verfahren zum Herstellen einer Beschichtung auf einem Substrat, welches mindestens folgende Schritte umfasst:

   i) Aufbringen einer zweikomponentigen Beschichtungszusammensetzung (2K-Beschichtungsmittel) gemäß einem der Ansprüche 1 bis 5 auf mindestens einen Teil eines zu beschichtenden Substrats und
   ii) Härten der Beschichtungszusammensetzung aus Schritt i).

7. Substrate beschichtet mit einer Beschichtung, erhältlich nach einem Verfahren gemäß Anspruch 6.

8. Verfahren zum Herstellen einer Beschichtung auf einem Substrat gemäß Anspruch 6, oder Substrate beschichtet mit einer Beschichtung gemäß Anspruch 7, **dadurch gekennzeichnet, dass** es sich bei der Beschichtung um einen Klarlack handelt.

9. Verfahren gemäß Anspruch 8, oder Substrate gemäß Anspruch 8, **dadurch gekennzeichnet, dass** es sich bei dem Klarlack um einen Reparaturklarlack, im Besonderen für Fahrzeuge handelt.

10. Verwendung einer zweikomponentigen Beschichtungszusammensetzungen (2K-Beschichtungsmittel) gemäß einem der Ansprüche 1 bis 5 zur Herstellung von wasserstabilen Beschichtungen.

Europäisches Patentamt
European Patent Office
Office européen des brevets

# EUROPÄISCHER RECHERCHENBERICHT

Nummer der Anmeldung
EP 19 15 8880

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2014/151307 A1 (BAYER MATERIALSCIENCE LLC [US]; SQUILLER EDWARD P [US] ET AL.) 25. September 2014 (2014-09-25) * Absatz [0036]; Beispiel 1 * | 1-10 | INV. C08G18/79 C09D175/00 C08G18/38 C07C227/06 |
| Y,D | EP 0 816 326 A1 (BAYER AG [DE]) 7. Januar 1998 (1998-01-07) * Anspruch 1; Beispiele 6,7 * | 1-10 | |
| Y,D | EP 1 197 507 A2 (BAYER AG [DE]) 17. April 2002 (2002-04-17) * Absatz [0012]; Beispiele 3,4 * | 1-10 | |
| Y | DE 10 2009 010068 A1 (BAYER MATERIALSCIENCE LLC [US]) 8. Oktober 2009 (2009-10-08) * Anspruch 1 * | 1-10 | |
| Y | US 2014/272162 A1 (OLSON AHREN [US]) 18. September 2014 (2014-09-18) * Ansprüche 1,9 * | 1-10 | RECHERCHIERTE SACHGEBIETE (IPC) |
| Y | WO 2010/034109 A1 (QUANTUM TECHNICAL SERVICES LTD [CA]; LAGRANGE TONY [CA] ET AL.) 1. April 2010 (2010-04-01) * Tabelle 5 * | 1-10 | C08G C09D C07C |
| Y | WO 2019/034586 A1 (COVESTRO DEUTSCHLAND AG [DE]) 21. Februar 2019 (2019-02-21) * Seite 13, Zeilen 27,28; Beispiel 5 * | 1-10 | |
| Y | EP 3 061 622 A1 (SEAGRAVE COATING CORP [US]) 31. August 2016 (2016-08-31) * Absatz [0017]; Tabellen 1-4 * | 1-10 | |
| Y | US 2012/183692 A1 (BECKER IV JOHN C [US]) 19. Juli 2012 (2012-07-19) * Tabellen 1-3 * | 1-10 | |
| | -/-- | | |

Der vorliegende Recherchenbericht wurde für alle Patentansprüche erstellt

1

| Recherchenort | Abschlußdatum der Recherche | Prüfer |
|---|---|---|
| München | 11. Juli 2019 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
&: Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 19 15 8880

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| T | WO 2019/057626 A1 (COVESTRO DEUTSCHLAND AG [DE]) 28. März 2019 (2019-03-28)<br>* Anspruch 1 *<br>----- | 1-10 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 11. Juli 2019 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 19 15 8880

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-07-2019

| Im Recherchenbericht angeführtes Patentdokument | Datum der Veröffentlichung | Mitglied(er) der Patentfamilie | Datum der Veröffentlichung |
|---|---|---|---|
| WO 2014151307 A1 | 25-09-2014 | CN 105073815 A | 18-11-2015 |
| | | EP 2970556 A1 | 20-01-2016 |
| | | US 2016024339 A1 | 28-01-2016 |
| | | WO 2014151307 A1 | 25-09-2014 |
| EP 0816326 A1 | 07-01-1998 | AT 192734 T | 15-05-2000 |
| | | CA 2209131 A1 | 02-01-1998 |
| | | EP 0816326 A1 | 07-01-1998 |
| | | ES 2148877 T3 | 16-10-2000 |
| | | JP 4173567 B2 | 29-10-2008 |
| | | JP H1087583 A | 07-04-1998 |
| | | US 5821326 A | 13-10-1998 |
| EP 1197507 A2 | 17-04-2002 | AT 296845 T | 15-06-2005 |
| | | CA 2358760 A1 | 11-04-2002 |
| | | DE 10050137 A1 | 18-04-2002 |
| | | EP 1197507 A2 | 17-04-2002 |
| | | ES 2242691 T3 | 16-11-2005 |
| | | JP 4355460 B2 | 04-11-2009 |
| | | JP 2002121178 A | 23-04-2002 |
| | | MX PA01010297 A | 10-11-2004 |
| | | PT 1197507 E | 30-09-2005 |
| | | US 2002132965 A1 | 19-09-2002 |
| DE 102009010068 A1 | 08-10-2009 | CA 2657014 A1 | 05-09-2009 |
| | | DE 102009010068 A1 | 08-10-2009 |
| | | US 2009226644 A1 | 10-09-2009 |
| US 2014272162 A1 | 18-09-2014 | KEINE | |
| WO 2010034109 A1 | 01-04-2010 | KEINE | |
| WO 2019034586 A1 | 21-02-2019 | EP 3444289 A1 | 20-02-2019 |
| | | WO 2019034586 A1 | 21-02-2019 |
| EP 3061622 A1 | 31-08-2016 | CA 2919804 A1 | 03-08-2016 |
| | | EP 3061622 A1 | 31-08-2016 |
| | | US 2016237309 A1 | 18-08-2016 |
| US 2012183692 A1 | 19-07-2012 | KEINE | |
| WO 2019057626 A1 | 28-03-2019 | EP 3456755 A1 | 20-03-2019 |
| | | WO 2019057626 A1 | 28-03-2019 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0403921 A
- EP 0639628 A
- EP 0667362 A
- EP 0689881 A
- US 5214086 A
- EP 0699696 A
- EP 0596360 A
- EP 0893458 A
- DE 19701835
- US 5243012 A
- EP 0470461 A
- WO 15130501 A
- WO 15130502 A
- EP 0816326 A
- EP 1197507 A
- DE 102006002153
- WO 2018074884 A
- WO 05058996 A
- WO 9422968 A
- EP 0276501 A
- EP 0249201 A
- WO 9712945 A


### In der Beschreibung aufgeführte Nicht-Patentliteratur


- **HAUBEN WEYL.** *Meth. d. Org. Chemie,* 1957, vol. 11/1, 272
- *Usp. Khim.,* 1969, vol. 38, 1933
- **A. VALET.** Lichtschutzmittel für Lacke. Vincentz Verlag, 1996